(19) **Europäisches Patentamt**

**European Patent Office**

**Office européen des brevets**

(11) **EP 1 207 334 B1**

(12) **EUROPÄISCHE PATENTSCHRIFT**

(45) Veröffentlichungstag und Bekanntmachung des Hinweises auf die Patenterteilung:
**14.06.2006 Patentblatt 2006/24**

(51) Int Cl.:
*F16M 11/04* (2006.01)      *G02B 7/00* (2006.01)

(21) Anmeldenummer: **01125606.2**

(22) Anmeldetag: **26.10.2001**

(54) **Stativ**

Stand

Support

(84) Benannte Vertragsstaaten:
**DE FR GB**

(30) Priorität: **12.11.2000 DE 20019105 U**

(43) Veröffentlichungstag der Anmeldung:
**22.05.2002 Patentblatt 2002/21**

(73) Patentinhaber: **Leica Microsystems AG**
**9435 Heerbrugg (CH)**

(72) Erfinder: **Metelski, Andrzej**
**8590 Romanshorn (CH)**

(74) Vertreter: **Reichert, Werner Franz**
**Leica Microsystems AG,**
**Corporate Patents + Trademarks Department,**
**Ernst-Leitz-Strasse 17-37**
**35578 Wetzlar (DE)**

(56) Entgegenhaltungen:
| | |
|---|---|
| **EP-A- 0 700 665** | **EP-B- 0 433 426** |
| **WO-A-99/01693** | **DD-A- 221 571** |
| **DE-A- 3 739 080** | **DE-A- 19 742 050** |
| **DE-U- 20 019 105** | **DE-U- 20 019 106** |
| **DE-U- 20 019 107** | **FR-A- 2 605 757** |
| **US-A- 4 515 333** | **US-A- 5 253 832** |
| **US-A- 5 397 323** | **US-A- 6 070 839** |
| **US-A- 6 129 319** | |

**Beschreibung**

Die Erfindung betrifft ein Stativ, insbesondere für Operationsmikroskope. Operationsmikroskope müssen über einen vorgegebenen Bereich leicht schwenkbar sein und sollten die danach eingestellte Position beibehalten. Aus diesem Grund sind bei einer Gruppe von bekannten Stativen Ausgleichsgewichte vorgesehen, die das Gewicht des Mikroskops und seiner Zusatzeinrichtungen kompensieren. Am häufigsten werden die Ausgleichsgewichte balkenwaagenartig angeordnet. Besondere Ausbildungsformen solcher Balkenwaagenanordnungen sind beispielsweise der Aufbau "OHS™" der Anmelderin, bei dem über Parallelogrammträger Ausgleichsgewichte von oben nach unten verlegt sind, sodass der Gesamtschwerpunkt des Stativs im unteren Bereich des Stativaufbaus liegt. Der Prinzipaufbau des OHS™ ist in der internationalen Patentanmeldung WO 97/13997 (1997) und der korrespondierenden US 6 129 319 symbolisch dargestellt.

Der Gewichtsausgleich für die leichte Bedienbarkeit eines Mikroskops bzw. Beweglichkeit desselben im Raum und für die diesbezügliche Kompensation von Gewichtsänderungen am Mikroskop durch Hinzugeben oder Wegnehmen von Zusatzeinrichtungen am Mikroskop erfolgt bei einem bekannten Stativ "MS 1" der Anmelderin über eine Druckfeder, die einen Parallelogrammträger diagonal abstützt. Dieser Parallelogrammträger dient beim Aufbau MS 1 als schwenkbarer horizontaler Träger für das Mikroskop. Die besondere Parallelträgerkonstruktion wurde durch die Anmelderin in der europäischen Patentanmeldung EP 433426 A1 (WO 91/472) veröffentlicht. In der internationalen Patentanmeldung WO 99/1693 (1999) ist der Aufbau des MS 1 symbolisch dargestellt.

Für ein verbessertes Kippverhalten des Stativs sieht das MS 1 als Ausgleichsgewicht einen Schaltkasten vor, der sowohl die elektrische Stromversorgung für das Mikroskop, als auch seine Beleuchtungseinrichtung, seine Steuerungen o.dgl. und gegebenenfalls ein Zusatzgewicht enthält. Der Schaltkasten ist starr an der vertikalen Tragsäule des Stativs montiert und übernimmt dort lediglich einen Gewichtsausgleich um die vertikale Achse der senkrechten Tragsäule im Hinblick auf eine Verbesserung des Kippmomentes des Stativs.

In der DE 19742050 A1 (1999) wird Bezug genommen auf einen Aufsatz "Gewichtsausgleich an feinmechanischen Geräten" von H. Hilpert in Heft 2/1965 der Zeitschrift Feingerätetechnik, 14. Jahrgang.

In diesem Artikel aus dem Jahr 1965 wird auf verschiedene gewichtskompensatorische Massnahmen in der Feingerätetechnik eingegangen, die in erster Linie nicht durch Gegengewichte, sondern durch federkompensatorische Massnahmen (wie vergleichsweise auch bei der Parallelträgerkonstruktion des MS 1) erzielt werden.

Die DD 221571 A1 (1985) zeigt einen Stativaufbau mit einem Hebelarm, der durch eine Feder, die über einen Seilzug mit dem Hebelarm verbunden ist, Gewicht kompensiert. Am distalen Ende des Hebelarms befindet sich das Operationsmikroskop. Die Grundjustierung dieses Operationsmikroskops erfolgt über eine Gewindespindel, mit der das gehäusefeste Ende der Feder weiter vom Hebelarm weggezogen oder näher zu ihm hingeführt wird. Gewichtsänderungen am Mikroskop werden dadurch kompensiert, dass der Anlenkpunkt des Seilzugs relativ zum Hebelarm über eine Spindel verstellt wird.

Um in allen möglichen Winkellagen des Stativs ein gleichmässiges Gegenmoment zu erzielen, ist es erforderlich, dass sich der erwähnte Angriffspunkt des Seilzugs auf einer Verbindungslinie zwischen der Drehachse des Hebelarms und dem Masseschwerpunkt des Mikroskops befindet. Dies wird durch das Betätigen einer Verstelleinrichtung in Form einer Schnecke erreicht, die eine mit dem Hebelarm verbundene Scheibe um die Drehachse des Hebelarms dreht.

Bei diesem Aufbau sind somit eine Fülle von Verstellmassnahmen erforderlich, um den gewünschten Effekt zu erzielen. Abgesehen davon, zwingt die Konstruktion dieses bekannten Aufbaus zu einem hohen Gesamtschwerpunkt des Stativs, da sämtliche Gewichtsausgleichsvorrichtungen oberhalb des Mikroskops angeordnet sind.

Die DE 3739080 A1 (1989) gibt ebenso eine Federvorrichtung zum Gewichtsausgleich für Stative an, bei der Seilzüge in Kombination mit Federn zu einem Gewichtsausgleich führen sollen. Dabei geht es um die Kraftunterstützung einer Verstellbewegung, die eine Bedienperson an einem Handgriff ausübt. Es geht dort allerdings nicht darum, eine Last in einem "schwebenden Zustand" zu halten, wie dies bei Operationsmikroskopen gewünscht ist.

Die US 5 253 832 beschreibt ein Stativ mit einem Ständer und einem daran schwenkbar angeordneten Tragarm für eine Last. Zum Gewichtsausgleich und zur Balancierung ist der Tragarm über einen Seilzug mit einer Feder verbunden. Über die Seilzug-Feder-Konstruktion wird jedoch keine Verbesserung der Kippsicherheit erreicht.

Die FR 2 605 757 beschreibt einen fotografischen Reflektor mit einem Stativ. Das Stativ ist mit einer Seilzug-Feder-Konstruktion zum Gewichtsausgleich ausgestattet. Auch hier trägt die Seilzug-Federkombination nicht zur Verbessung der Kippsicherheit bei.

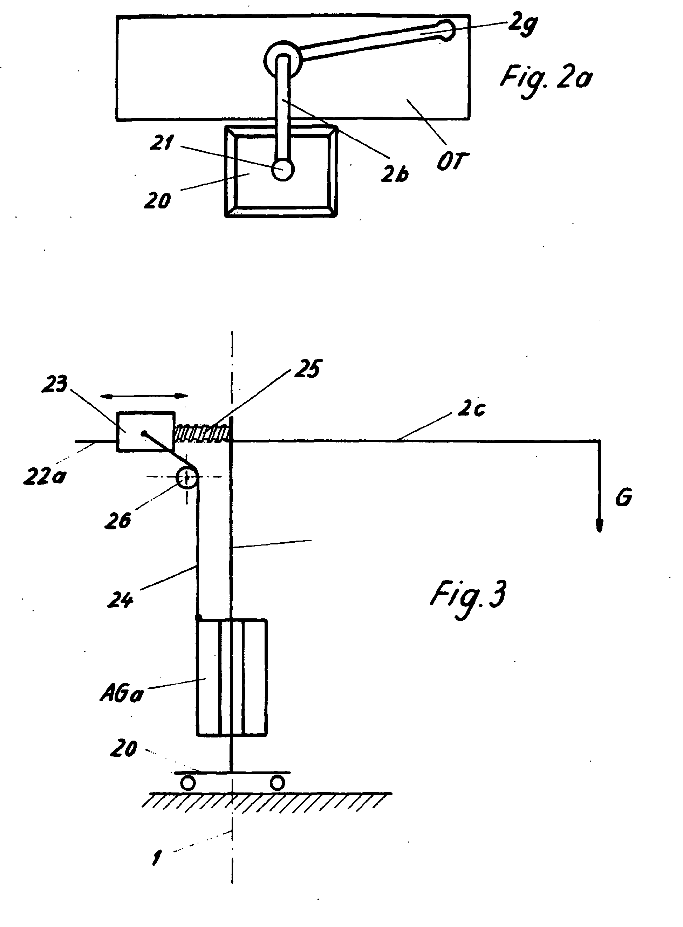

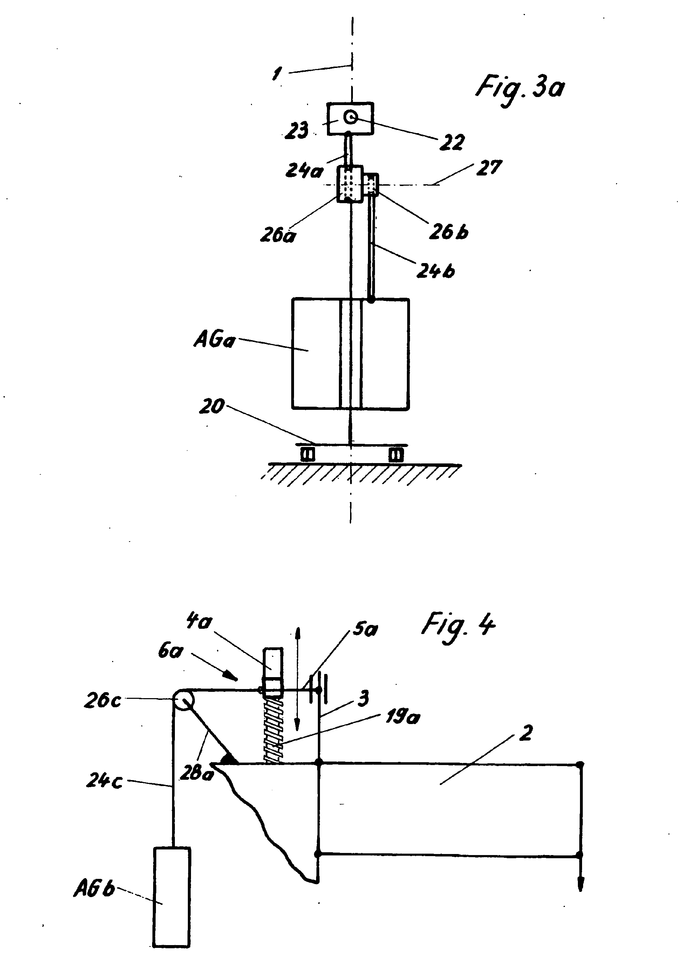

Demgegenüber ist in der US 5397323 (1992) ein Operationsroboter mit Parallelogrammträgern vorgestellt, bei dem unter anderem das Gewicht des Instruments über einen Seilzug mittels Gegengewicht gewichtskompensiert gehalten wird. Der Seilzug ist dabei geschlossen aufgebaut, d.h. über eine obere und untere Umlenkrolle wird je ein Seil vom Instrument bis zum Gegengewicht geführt (Fig.3 der US -323).

Ein solcher Aufbau setzt voraus, dass das Gegengewicht in unmittelbarer Nähe des Instruments angebracht ist. Er wäre daher für den Einsatz an einem Operationsmikroskop nur schlecht anwendbar.

Die DE 19742050 A1 (1999) offenbart einen Stativaufbau mit einem schwenkbaren Parallelogrammträger, der über einen Seilzug und eine Gewichtsausgleichsfeder so gewichtskompensiert ist, dass die zusätzlich vorhandenen Ausgleichsgewichte, die nach dem oben erwähnten Waagenprinzip wirken, besonders klein ausgebildet sein können. Bei diesem Aufbau wird der Seilzug in besonderer Form geführt, um den durch den endlichen Umlenkradius bedingten Gewichtsausgleichsfehler in einem weiten Schwenkbereich des Schwenkarms zu minimieren. Der Gewichtsausgleichsfehler wird durch diese Massnahme jedoch nicht eliminiert, sodass bei bestimmten Schwenkpositionen nach wie vor ein Verstellen der Ausgleichsgewichte erforderlich ist, um die gewünschte Balancierung zu erreichen.

Die US 6070839 (2000) offenbart einen weiteren Aufbau mit einem Schwenkarm und einer Seilzug-Feder-Konstruktion, die einen reinen Gewichtsausgleich - im Sinne des oben erwähnten diagonalen Parallelträgerarmausgleichs beim MS1 - ermöglicht, ohne jedoch auch Ausgleichsmomente zu einer Verbesserung der Kippsicherheit beizutragen. Im Falle von Gewichtsänderungen wird der Anlenkpunkt des Seilzugs, vergleichbar dem Aufbau in der erwähnten DD 221571, über eine Spindel verschoben.

Die US 5253832 (1999) beschreibt ein Stativ mit einer zentral angeordneten Zugfeder für den Gewichtsausgleich. Dieser Aufbau bietet keine einfache Verstellmöglichkeit für geänderte Lasten. Die Zugfeder selbst hat ein geringes Eigengewicht, sodass sie zwar dem Gewichtsausgleich, jedoch nicht der Balance um die vertikale Mittelachse (Kippsicherheit) dient.

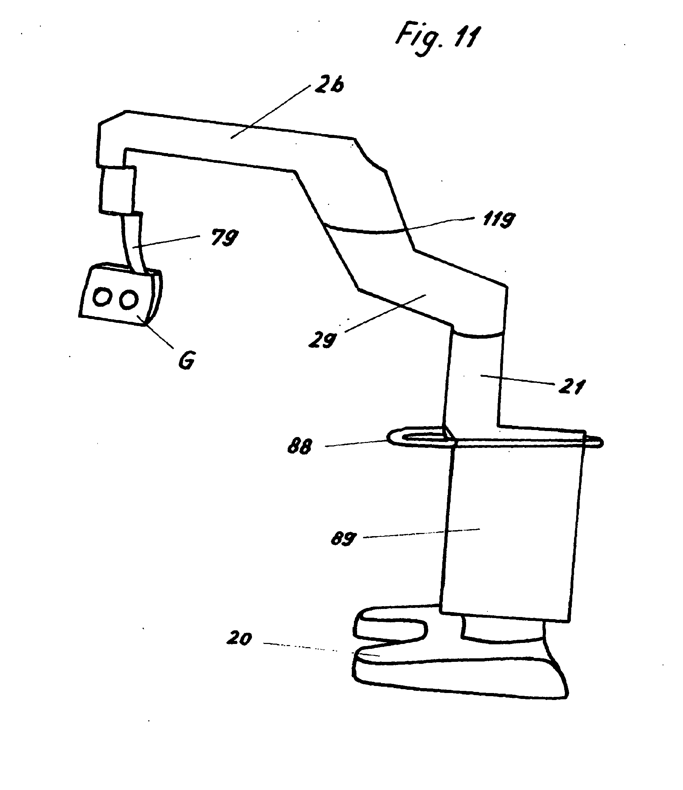

In der EP 700665 A1 (1995) ist des Weiteren ein Stativaufbau angegeben, der im Wesentlichen über Hebel und Hebelwinkelarme für einen Gewichtsausgleich sorgt. In der Fig.13 und 14 dieser veröffentlichten Patentanmeldung ist ein Bewegungsübertragungsmechanismus 157 vorgestellt, der Bewegungen des Mikroskopkörpers um Bewegungsachsen auf einen Schwenkarm überträgt. Durch diesen sehr komplizierten Aufbau mit vielen Teilen wird zwar ein gewisser Gewichtsausgleich geschaffen, andererseits ist jedoch eine Vielzahl von Hebeln und Winkelhebeln erforderlich, da der Gewichtsausgleich schlussendlich an solchen Hebeln montiert ist.

Bei dem MS 1 der Anmelderin, sowie bei verschiedenen Aufbauten anderer bekannter Stative und bei den oben angegebenen Stativen ist der Hauptträger, der unmittelbar die Last aufnimmt, oftmals nicht direkt an der vertikalen Ständersäule des Stativs befestigt, sondern an einem von dieser Säule abragenden zusätzlichen Horizontalarm. Die Kippsicherheit wird bei diesen Aufbauten in erster Linie durch eine entsprechende Ausbildung des Stativfusses erzielt, der entsprechend gross und schwer sein muss. Die Gewichtskompensation wird, wie schon weiter oben angegeben, durch eine Feder diagonal im Parallelogrammträger oder andere oben angegebene Massnahmen bewirkt.

Eine Verbesserung der Kippsicherheit und ein gewisser Ausgleich zur Kippverhinderung ist zwar - wie oben angegeben - gegebenenfalls durch die besondere Anordnung eines Schaltkastens o.dgl. möglich.

Aus der US 4 515 333 ist ein Stativ für Operationsmikroskop bekannt, das eine Ständersäule und einen daran schwenkbaren Tragarm aufweist. Zur Balancierung des Tragarms ist an einem Ende ein Seilzug befestigt, der an seinem anderen Ende mit einer Feder verbunden ist. Durch Schwenken des Tragarms in einer Vertikalebene, wird die Feder über den Seilzug mehr oder weniger gespannt. Abgesehen davon, dass derartige Federn nur in einem relativ kleinen Bereich konstant arbeiten, trägt die Feder aufgrund ihres geringen Gewichts auch hier nicht zu einer Verbesserung der Kippsicherheit bei.

Gegenüber den bekannten Aufbauten liegt der Erfindung die Aufgabe zugrunde, ein neuartiges Stativ für Operationsmikroskope zu schaffen, das kippsicherer ist, möglichst wenig Volumen aufweist und über eine optimale Gewichtskompensation verfügt. Unter Gewichtskompensation ist einerseits die Kompensation des Gewichts der Last zu verstehen, andererseits aber auch die Kompensation allfälliger Änderungen dieses Gewichts.

Gelöst wird diese Aufgabe erstmals durch ein System, bei dem über einen Seilzug und wenigstens eine Umlenkrolle eine konstante Ausgleichskraft, z.B. ein Gewicht für den Gewichtsausgleich eingesetzt ist, wobei das Gewicht entweder in der Nähe der Ständersäule, z.B. auf der der Last gegenüberliegenden Seite, konzentrisch zur Ständersäule oder bevorzugt innerhalb der Ständersäule untergebracht ist.

Damit liegt der Gesamtschwerpunkt des Stativs sehr tief und ist in Richtung Ständersäule bzw. Stativfuss legbar, sodass das Kippverhalten schon aus diesem Grund verbessert wird.

Durch den erfindungsgemässen Seilzug und die wenigstens eine Umlenkrolle ist es zudem möglich, das Ausgleichsgewicht an nahezu beliebigen Stellen anzubringen, bzw. den Seilzug über verschiedenst ausgebildete Tragarme (Horizontalarm) oder Tragarmteile zu führen, ohne nennenswerten Platzbedarf und ohne nennenswerte zusätzliche Gewichte über den Seilzug.

Es ist zwar schon ein Bodenstativ bekannt geworden (Standard und Universal), das über eine Umlenkrolle ein Kunststoffband einsetzt, um ein Ausgleichsgewicht in einer Ständersäule mit einem Ring um die Ständersäule zu verbinden, der einen waagrecht abragenden Arm stützt, an dem die Last montierbar ist. Dieser Aufbau erlaubt keinen Lastausgleich bei einem schwenkbaren Träger und insbesondere erlaubt er keinen Ausgleich bei einem Träger, der in sich in einer Horizontalebene schwenkbar ist (um die Ständersäule und um eine weitere Achse schwenkbar). Da das Ausgleichsgewicht bzw. die Bänder nicht am Schwenkarm direkt angreifen, wird die Ständersäule bzw. die Anlenkung des Tragarms an der Ständersäule stark auf Verkantung (Drehmoment des Armes an der Ständersäule) belastet. Ausserdem erfordert der bekannte Aufbau bei einer Gewichtsänderung an der Last eine Änderung des Ausgleichsgewichtes durch Hinzugeben oder Hinwegnehmen von Gewichten. Tut man das nicht, muss man den Ring an der Ständersäule fixieren, um ein Driften zu verhindern. Ein automatischer Balancierausgleich ist bei diesem bekannten Aufbau somit insgesamt nicht möglich.

Bei Weiterbildungen der Erfindung zeigt es sich als zweckmässig, wenn der Seilzug drehentkoppelt ist, d.h. wenn bei einem Schwenken des Tragarmes in einer horizontalen Ebene das Seil nicht auf Torsion belastet wird, bzw. sich allfällige Torsionsbelastungen an den Drehentkoppelstellen lösen. Alternativ oder zusätzlich können auch Seile verwendet werden, deren Aufbau gegenüber Torsionsbelastungen tolerant ist. Solche Seile sind insbesondere beispielsweise links-rechts-geflochtene Seile, oder Kombinationen aus links- und rechtsgeflochtenen Seilen.

Eine besondere Weiterbildung der Erfindung sieht vor, das Ausgleichsgewicht über eine Flaschenzugseilrolle aufzuhängen, sodass es zu einer Kraftübersetzung kommt und das Gewicht des Ausgleichsgewichts besonders hoch gewählt werden kann, was zu einer verbesserten Standfestigkeit des Stativs aufgrund des besonders tiefliegenden Schwerpunktes führt.

Umgekehrt liegt im Rahmen der Erfindung eine besondere Ausbildung, bei der mit Hilfe eines umgekehrten Flaschenzugsystems das Ausgleichsgewicht nur halb so gross dimensioniert werden kann, indem die Übersetzung beim Ausgleichsgewicht zu einem vergrösserten Weg, aber zu einer vergrösserten resultierenden Kraft für den Gewichtsausgleich führt.

Abgesehen von einem Flaschenzug sind erfindungsgemäss beliebige Übersetzungsvarianten über ein Rollengetriebe möglich, bei dem der Seilzug unterbrochen ist und an der Unterbrechungsstelle einerseits an einer Rolle mit grösserem Durchmesser angreift und andererseits das andere Ende des anderen Stückes des Seilzugs an einer mit dieser Rolle starr verbundenen kleineren Rolle angreift, sodass es zu einer Über- oder Untersetzung der Kraft des Ausgleichsgewichtes kommt. Anstelle von Rollen können auch vergleichbare Hebelgetriebe zum Einsatz kommen.

Eine andere Ausgestaltung der Erfindung sieht eine Art Waagebalken als Tragarm für die Last vor, der an seinem lastabgewandten Ende eine Führung aufweist, entlang der die Angriffsstelle eines Seilzugs (Gleitschuh) verschiebbar ist. Der Seilzug ist an seinem anderen Ende mit einem Ausgleichsgewicht verbunden, das im Bereich der Ständersäule in der Höhe frei schwebend angebracht ist.

Eine bevorzugte und einfache Konstruktion sieht eine an einem Ausgleichsarm verschiebbar gelagerte Rolle vor, die einen nicht verschiebbaren Bügel beaufschlagt, an dem der Seilzug angreift. Durch diese Konstruktion wird erreicht, dass bei einem Schwenken des Tragarms automatisch die proportionale Änderung der Angriffsstelle der Rolle am Bügel angepasst wird, sodass die gegeneinander wirkenden Momente in jeder Winkelposition die gleiche Proportion zueinander haben.

Bei Anwendung eines zusätzlichen Horizontalarmes, wie z.B. beim MS 1 wird so ein universell einsetzbares, über zwei Vertikalachsen schwenkbares Stativ mit ausgezeichnetem Gewichtsausgleich und verbesserter Kippsicherheit geschaffen.

In einer weiter Ausgestaltung der Erfindung ist das Ausgleichsgewicht entlang der Ständersäule gleit- oder rollgelagert oder reibungslos geführt. Auch kann wenigstens eine der Spindelverstellungen elektromotorisch, elektromagnetisch, hydraulisch und/oder pneumatisch antreibbar ausgebildet sein.

In einer vorteilhaften Weiterbildung der Erfindung ist der Antrieb mit einer Bremse für das Blockieren der Arme ausgestattet und/oder wenigstens eine der Spindelverstellungen computergesteuert ausgebildet.

Vorteilhaft kann an der vertikalen Ständersäule ein Geräteschrank mit zusätzlicher Gewichtsausgleichsfunktion angeordnet sein.

In einer weiteren Ausgestaltung ist ein Horizontalarm vorgesehen, der an der Ständersäule in einer Horizontalebene selbst schwenkbar ist und der den Tragarm in einer Horizontalebene schwenkbar lagert.

Außerdem ist es vorgesehen, dass wenigstens eine Umlenkrolle mittels einer Spindel in ihrer Relativposition zum gegebenenfalls verschiebbaren Anlenkpunkt des Seilzugs verstellbar ausgebildet ist.

Ferner kann es vorgesehen sein, dass das Ausgleichsgewicht aus wenigstens zwei Teilgewichten aufgebaut ist.

In einer weiteren Ausgestaltung der Erfindung sind in einem Seilzug wenigstens zwei parallele Seile vorgesehen, bei denen vorzugsweise die Wickelrichtung gegenläufig ausgebildet ist.

Anhand von beispielhaften Skizzen werden die Erfindung, Varianten und Weiterbildungen dazu näher erläutert. Es zeigen dabei

Fig.1 -   symbolisch einen theoretischen Aufbau mit mind. zwei vertikalen Achsen und einem Ausgleichsgewicht nach einem Waagenprinzip, das aus Gründen der zu geringen Kippsicherheit von der vorliegenden Erfindung verworfen wurde;

Fig.2 -   eine Variante des Aufbaus gemäss Fig.1, bei dem der Parallelogrammträger unmittelbar an der vertikalen Ständersäule befestigt ist; diese Variante ist durch die Erfindung ebenso verworfen, da sie den freien Arbeitsraum zu stark einschränkt;

Fig.2a - eine   Draufsicht auf einen Aufbau nach Fig.1 oder 2 in Arbeitsstellung;

Fig.3 und 3a -   einen erfindungsgemässen Aufbau nach dem Waagenprinzip;

Fig.4 und 5 -   Varianten eines erfindungsgemässen Aufbaus mit Tariergetriebe, jedoch ohne Cosinus-Ausgleich;

Fig.6 - eine Variante eines erfindungsgemässen Aufbaus mit Cosinusausgleich;

Fig.7 - einen Aufbau mit modifiziertem Tariergetriebe und Seilzug über einen Horizontalarm mit Cosinusausgleich;

Fig.8 - eine Variante des Aufbaus nach Fig.7 ohne Cosinusausgleich;

Fig.9 - eine Symboldarstellung des Flaschenzugs innerhalb des Seilzugs;

Fig.10 - den oberen Teil des Flaschenzugs in Schrägansicht und

Fig.11 - einen Stativaufbau mit einem Operationsmikroskop als Last.

Die Figuren werden übergreifend beschrieben, gleiche Bezugszeichen bedeuten gleiche Bauteile. Bezugszeichen mit gleichen Nummern, jedoch unterschiedlichen Indizes bedeuten geringfügig unterschiedliche Bauteile mit gleichen Aufgaben bzw. ähnlichen Wirkungen.

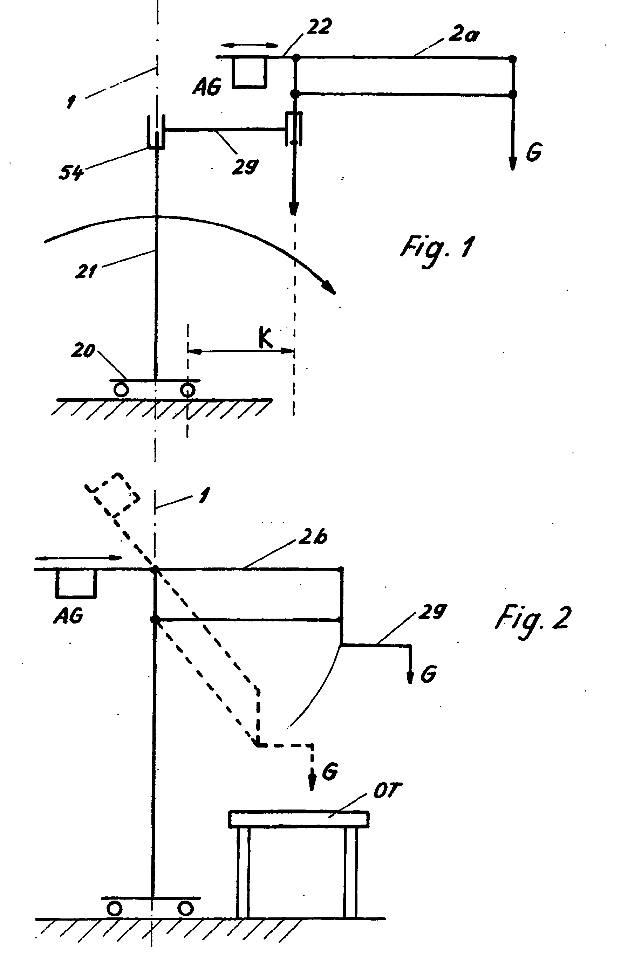

Fig.1 zeigt ein Beispiel einer denkbaren Gewichtsausgleichsmassnahme nach dem Waagenprinzip, die um die vertikale Achse 1 der Anbringungsstelle des Tragarms 2a erfolgen würde. Eine solche Massnahme könnte zunächst die Kippsicherheit des Stativs jedoch noch nicht verbessern, da die allfällig angebrachten Zusatzgewichte AG wegen des Horizontaltragarms 29 zunächst noch auf derselben Seite der vertikalen Ständersäule 21 sind, auf der auch die Last G angreift. (Kippmoment gemäss Pfeil über dem wirksamen Abstand k) Nur dann, wenn man den Ausgleicharm 22 entsprechend lang machen und ihn am Anlenkpunkt einbremsen würde, wobei er auch über die Achse der Ständersäule 21 ragen würde, könnte mit einem solchen Gewichtsausgleich gleichzeitig auch die Kippsicherheit verbessert werden. Dabei würde jedoch nachteiligerweise das Volumen des Gesamtaufbaus deutlich vergrössert werden. Beim Lösen der Bremse käme es ggf. zu einem Kippen. Abgesehen davon sind bei den herkömmlichen Stativen die Hauptträgerarme um ihre Montagepunkte nicht nur in der Höhe, sondern auch seitlich schwenkbar, wodurch Positionen (den Tragarm 2a um ca. 90° aus der Bildebene geschwenkt) entstehen können, bei denen auch bei verlängerten Waagenausgleichssystemen die gesamte Last auf einer Seite der Ständersäule 21 zu liegen kommt und daher die Kippgefahr wieder gross werden würde. Dieses müsste - wie in der Praxis versucht - durch einen ausreichend grossen oder schweren Stativfuss 20 ausgeglichen werden.

In Fig.2 sieht man eine Variante zum Aufbau nach Fig.1, bei der der Angriffsort des Parallelogrammträgers 2b an die vertikale Säule 21 des Stativs verlegt wurde. Hierdurch ist zwar das Kippverhalten verbessert, da das Ausgleichsgewicht AG auf der linken Seite der vertikalen Achse 1 liegt. Wie die Skizze jedoch andeutet, ist bei diesem Aufbau innerhalb des Kreises eine nur ungenügende Raumfreistellung für den Anwender die Folge. Der Parallelogrammträger 2b dringt in den Raum ein, der beim Aufbau gemäss Fig.1 freibliebe. Dies führt zu Behinderungen des Anwenders, z.B. bei der Arbeit an einem Operationstisch OT, wie symbolisch angedeutet.

In der Fig.2a sieht man die Draufsicht auf die Arbeitsstellung des Mikroskops gemäss Fig.1 oder 2.

Demgegenüber ist der grundsätzliche Aufbau eines erfindungsgemässen Stativs gemäss Fig.3 insofern unterschiedlich, als das Ausgleichsgewicht in Form einer vertikal frei beweglichen Ausgleichslast AGa ausgebildet ist, deren Angriffspunkt am waagenförmigen Ausgleichsarm 22a, der eine Verlängerung des Tragarms 2c darstellt, montiert ist. Ein Gleitschuh 23, an dem ein Seilzug 24 befestigt ist, ist mittels einer Spindel 25 entlang des Ausgleichsarmes verschiebbar. Wird der Gleitschuh 23 weiter nach links geschoben, erhöht sich der Effekt (Moment) des Ausgleichgewichtes AGa und die Last G kann grösser bemessen werden. Der Seilzug 24 könnte theoretisch eine direkte Verbindung zwischen dem Gleitschuh 23 und dem Ausgleichsgewicht AGa bilden. Bei einer solchen direkten Verbindung entsteht jedoch beim Verschwenken des Tragarms 2c ein Cosinusfehler im wirksamen Ausgleichsgewicht durch die Schrägstellung des Seilzugs 24.

In der dargestellten, verbesserten Ausbildungsform wirkt der Seilzug jedoch auf eine Umlenkrolle 26, deren Drehpunkt starr zur Stativsäule 21 festgelegt ist. Dies bewirkt, dass das Seil des Seilzugs 24 im Bereich des Ausgleichsgewichts AGa und darüber seine Parallelposition zur Ständersäule 21 nicht verlässt. In seinem Bereich oberhalb der Umlenkrolle 26 folgt das Seil des Seilzugs 24 jedoch dem Gleitschuh 23, sodass dort eine Schrägstellung des Seiles in Bezug auf die Ständersäule 21 möglich ist.

Fig.3a zeigt eine Variante dieser Umlenkrolle 26, die gleichzeitig als Übersetzung dient: Der Seilzug 24 ist bei dieser Variante zweigeteilt, wobei der obere Teil 24a einerseits mit einer Rolle 26a verbunden ist und andererseits am Gleitschuh 23 befestigt ist. Der Gleitschuh 23 gleitet am Ausgleichsarm 22, der durch die Ständersäule 21 gehalten ist. Eine kleinere Umlenkrolle 26b ist mit der grösseren 26a starr verbunden. An ihr ist der untere Seilzug 24b befestigt, bzw. aufgewickelt, der andererseits mit dem Ausgleichsgewicht AGa verbunden ist. Ein Verschieben des Gleitschuhs 23 entlang des Ausgleichsarms 22 führt somit zu einer Rotation der Umlenkrolle 26a und zu einer drehgleichen Rotation der Umlenkrolle 26b um die Drehachse 27.

Zumal der Radialabstand des Seilzugs 24b von der Drehachse 27 der Umlenkrolle 26b geringer ist als der Radialabstand des Seilzugs 24a zur Drehachse 27 der Umlenkrolle 26a, führt dies zu einer Untersetzung, d.h. das Ausgleichsgewicht AGa kann wesentlich grösser sein, als es sein müsste, wenn der Seilzug 24a direkt mit dem Seilzug 24b verbunden wäre. Für Fälle, bei denen das Ausgleichsgewicht kleiner sein soll, werden die beiden Rollen 26a und 26b vertauscht, woraus sich eine Übersetzung ergibt, bei der ein geringenres Ausgleichsgewicht eine grössere Ausgleichswirkung erzielt als im vorher beschriebenen Fall.

In den Fig.3 und 3a ist das Ausgleichsgewicht AGa symbolisch ringförmig um die Ständersäule 21 angeordnet. Im Rahmen der Erfindung sind jedoch insbesondere Lösungen vorgesehen, bei denen das Ausgleichsgewicht innerhalb der Ständersäule 21 oder auch nur auf der Seite der Ständersäule 21 angeordnet ist, die der Last G um die Achse 1 gegenüberliegt.

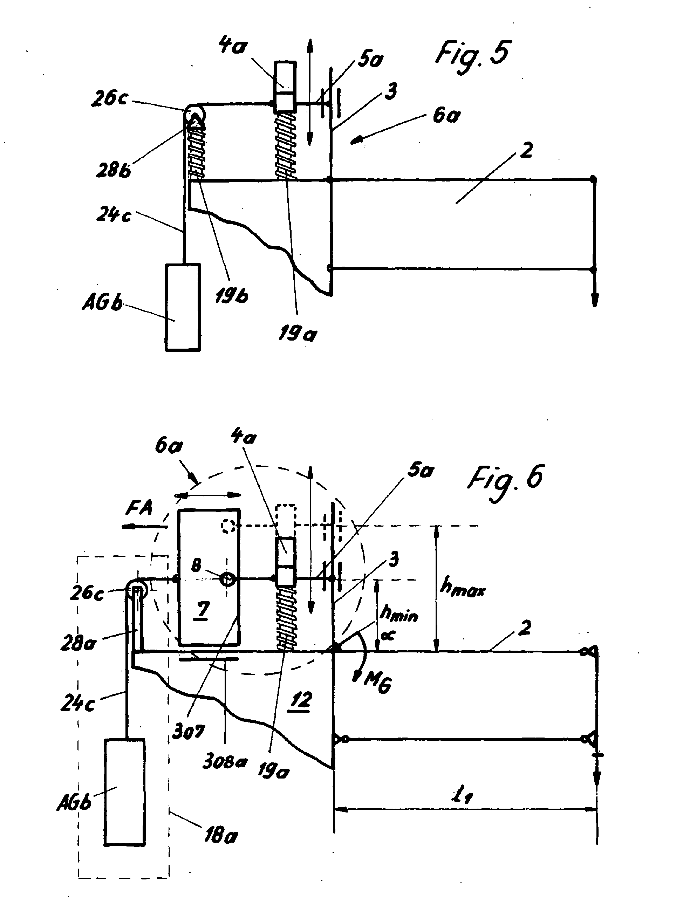

In Fig.4 sieht man einen Aufbau ohne Ausgleichsarm 22. Bei diesem Aufbau übernimmt ein Arm 3, der mit dem Tragarm 2 starr verbunden ist, die Ausgleichsfunktion. Parallel zu ihm ist ein Arm 4a vorgesehen, der eine Spindel 19a aufweist. Gelenkig mit den beiden Armen 3 und 4a ist ein Verbindungsarm 5a vorgesehen, der in seiner Höhenlage mittels der Spindel 19a verstellt werden kann. Mit dem Verbindungsarm 5a ist ein Seilzug 24c verbunden, der um eine Umlenkrolle 26c geführt ist und an seinem anderen Ende das Ausgleichsgewicht AGb trägt. Die Rolle 26c kann bei Bedarf entsprechend der Darstellung in Fig. 3a ebenfalls zweiteilig ausgeführt sein und eine Unter- oder Übersetzungsfunktion aufweisen.

Als Variante zum Aufbau der Fig.4 ist der Aufbau von Fig.5 zu verstehen, bei dem einerseits das als Tariergetriebe wirkende Parallelogramm 6a vorgesehen ist, andererseits aber anstelle einer einfachen Abstützung 28 gemäss Fig.4 für die Umlenkrolle 26c eine spindelgesteuerte Abstützung 28b vorgesehen ist, die es ermöglicht, parallel zur Verschiebung des Verbindungsarms 5a auch die Position der Umlenkrolle 26c zu verändern. Dies bedeutet zwar mehr mechanischen und bauteilmässigen Aufwand, verbessert allerdings das Ausgleichsverhalten der Gesamtanordnung.

Die Lastausgleichseinheit 18 ist in der Fig. 6 aus dem Seilzug 24c, der Umlenkrolle 26c und dem Ausgleichsgewicht AGb aufgebaut. Bei einer Verstellung der Spindel 19a kommt es zu einer Höhenverlagerung des Verbindungsarms 5a an der Wange eines Wagens 7, der relativ zum Grundkörper 12 seitlich verschiebbar ist. Über den Seilzug 24c, der mit dem Wagen 7 verbunden ist, wird die konstante Ausgleichskraft FA auf den Wagen und damit auf den Verbindungsarm 5a aufgebracht, der lediglich durch seinen Abstand zum Grundkörper 12 die entsprechende Übersetzung für Gewichtsänderungen bei der Last G mit sich bringt.

Mit G ist die Last, bzw. das Operationsmikroskop angegeben, das am distalen Ende eines schwenkbaren Parallelogrammträgers 2 gehalten ist. Der obere Arm des Parallelogrammträgers 2 ist mit einem Arm 3 starr verbunden, der mit einem weiteren parallelen Arm 4 und einem Verbindungsarm 5 zu einem weiteren Parallelogramm 6a verbunden ist.

Dieses Parallelogramm 6a bildet das Tariergetriebe, in dem der

Verbindungsarm 5a in seiner parallelen Lage höhenverstellbar ist, wodurch die Geometrie des Parallelogramms 6a mittels Spindel 19a veränderbar ist. Ein Verschwenken des Parallelogrammträgers 2 nach oben oder nach unten führt zu einem Verschwenken des Parallelogramms 6a nach links oder nach rechts.

Die gestrichelte Linie $h_{max}$ gibt die äusserste Lage des Verbindungsarms 5a an, bei der die grösste Hebelarmübersetzung besteht, d.h. bei der G die grösste Last annehmen kann. Beim Verschwenkvorgang des Parallelogrammträgers 2 verändert sich die Geometrie des Parallelogramms 6a woraus sich ein automatischer Kompensationseffekt (Cosinusausgleich) bei den Ausgleichskräften ergibt.

Dies nach der Formel M(G)=M(F) bzw. l1x G = h1 x F bzw. l2 x G = h2 x F, wobei M(G) das Moment der Last und M(F) das Moment der Gegenkraft angibt.

$$l2 = l1 \times \cos \alpha \qquad h2 = h1 \times \cos \alpha \qquad \frac{l2}{h2} = \frac{l1.\cos a}{h1.\cos a} = \frac{l1}{h1} \ .$$

Im Falle einer Gewichtsänderung an G muss man somit erfindungsgemäss lediglich den Verbindungsarm 5a parallel entlang dem Arm 3 verschieben, um dementsprechend $h_{min/max}$ zu ändern, damit bei konstanter Ausgleichskraft FA die Kompensation der Gewichtsänderung erzielt wird.

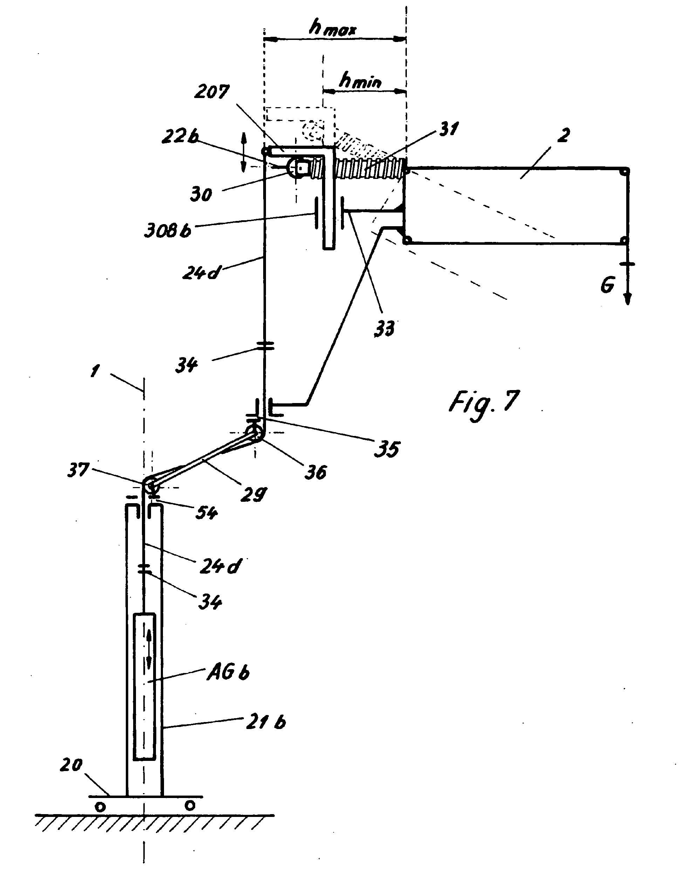

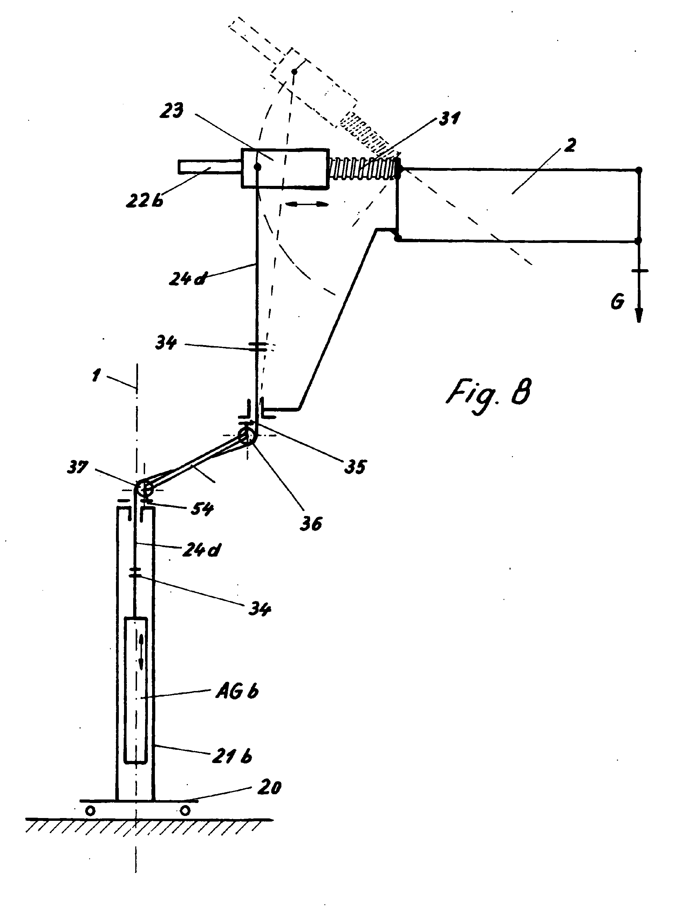

In den Fig.4 bis 6 ist das Gewicht AGb freihängend dargestellt. In der Praxis wird dieses natürlich in einem Käfig, an einer Führungsstange oder in der Ständersäule des Stativs untergebracht sein, wie dies beispielsweise in den Fig.7 und 8 angedeutet ist.

Der Aufbau gemäss Fig.7 unterscheidet sich von den Fig.3 bis 6 grundsätzlich durch einen zusätzlichen Horizontalarm 29, der den Tragarm 2 um eine vertikale und um eine horizontale Achse schwenkbar trägt. Zwischen dem Horizontalarm 29 und dem Tragarm 2 ist eine andere Form des Tariergetriebes vorgesehen, als bisher beschrieben: Eine Stützrolle 30 ist mittels einer Spindel entlang eines Ausgleichsarms 22b verschiebbar. Es ist dabei unwesentlich, ob die Rolle 30 auf einer Mutter sitzt, oder ob sie auf einem Gleitschuh gelagert ist, der auf einer Stange (Verlängerung des oberen Arms des Tragarms 2) mittels paralleler Gewindespindel längsverschiebbar ist.

Ein Bügel 207 ruht auf der Rolle 30 und ist einerseits in einer gehäusefesten Parallelführung 33 des Tariergetriebes geführt. Andererseits ist er mit dem Seil des Seilzugs 24d verbunden. Kommt es zu einer Längsverstellung der Rolle 30 durch die Spindel 31, so führt dies zu einem unterschiedlichen Ausgleichsgewichtsangriff über den Seilzug 24d am Ausgleichsarm 22b. An der Stelle 34 ist der Seilzug 24 drehentkoppelt, damit der Tragarm 2 in einer Horizontalebene um ein Lager 35 schwenken kann.

Entlang des Horizontalarmes 29 sind zwei weitere Umlenkrollen 36 und 37 vorgesehen, die den Seilzug 24d in das Innere der Ständersäule 21b führen. Im Inneren der Ständersäule 21 b befindet sich das Ausgleichsgewicht AGb, das vorzugsweise nochmals über eine Drehkopplung 34 drehentkoppelt ist. Das Ausgleichsgewicht AGb ist vorzugsweise aus Transportgründen und zur erleichterten Vorortmontage wenigstens zweiteilig ausgebildet.

Wie ersichtlich, benötigt dieser Aufbau nur minimal Platz. Andererseits ist das relativ schwere Ausgleichsgewicht AGb symmetrisch um die vertikale Achse 1 der Ständersäule 21 b verteilt und bewirkt vorteilhafterweise eine Absenkung des Gesamtschwerpunktes in Richtung Stativfuss 20.

Verschiedene Bezugszeichen in Fig.7 haben folgende Entsprechungen. Der Ausgleichsarm 22b entspricht etwa dem Arm 3 aus Fig.6.

Die Spindel 31 übt etwa die gleiche Funktion aus wie 19a aus Fig.6. Der Bügel 207 hat eine vergleichbare Funktion wie der Wagen 7 bzw. seine Wange 307 aus Fig.6 und die Rolle 30 ist etwa funktionsgleich mit der Rolle 8 aus Fig.6. Die wirksamen Längen $h_{max}$-$h_{min}$ am Ausgleichsarm 22b entsprechen den Längen $h_{max}$-$h_{min}$ am Arm 3 aus Fig.6. Bedingt durch die Führung 308a (Fig.6), bzw. 308b (Fig.7), ist die Cosinuskorrektur bei beiden Aufbauten sichergestellt.

Der Aufbau von Fig.8 ist ein vereinfachter Aufbau gemäss Fig.7 und verzichtet auf die Rolle 30, sowie auf den Bügel 207. Stattdessen wird lediglich der Befestigungspunkt des Seilzugs 24d an einem Gleitschuh 23 festgelegt, der über die Spindel 31 entlang des Ausgleichsarms 22b verschiebbar ist. Bei Bedarf kann eine der Rollen 36, 37 als Getrieberolle entsprechend dem Aufbau gemäss Fig.3a ausgebildet sein, um eine Über- oder Untersetzung am Seilzug 24d zu bewirken.

Da zwischen den $h_{min}$- und $h_{max}$-Einstellungen eine unterschiedliche Auswirkung der Seilzugsangriffsrichtung am Gleitschuh 23 auftritt, kommt es zu keinem ausreichenden Cosinusausgleich. Dies führt u.U. dazu, dass je nach Schwenkstellung des Tragarms 2 eine variable Kompensationswirkung durch das Ausgleichsgewicht AGb auftritt, so dass der Anwender eine Unbalance bemerken kann, weshalb dieser Aufbau nicht bevorzugt ist. Andererseits ist ein solcher erfindungsgemässer Aufbau jedoch gegenüber einem Aufbau mit diagonaler Federabstützung bei gleicher Kippsicherheit mit einer grösseren Ausladung produzierbar.

Zudem ist ein solcher Aufbau besonders preisgünstig herzustellen.

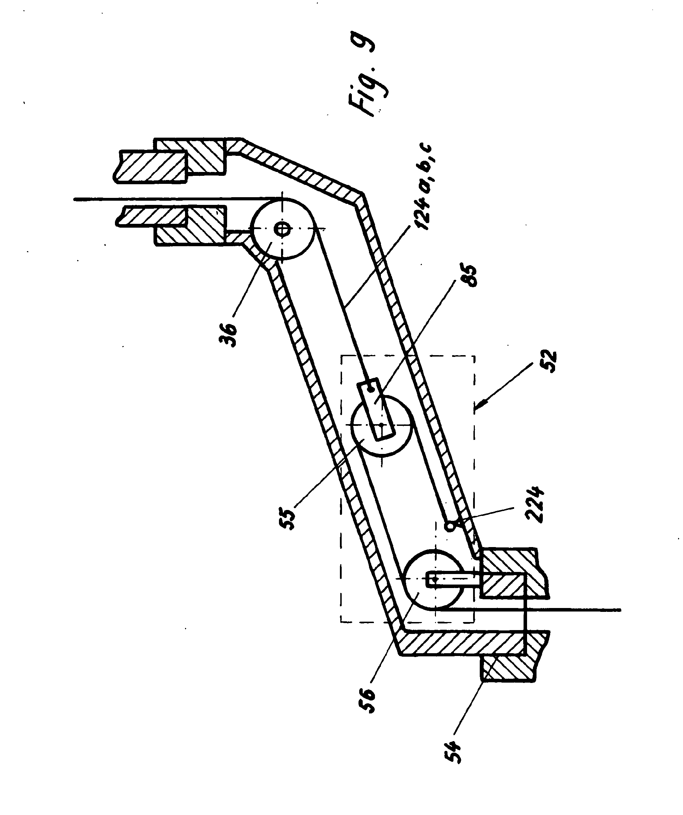

In Fig.9 sieht man einen erfindungsgemäss aufgebauten Seilzug mit einem einfachen Flaschenzug. Dieser umfasst eine Rolle 55, die zwischen den Rollen 36 und 37 beweglich positioniert ist. Die Seilzüge 24a-c umschlingen die Rolle 55 und sind bei 424 am Gehäuse befestigt. Bei diesem Aufbau ergibt sich somit eine Übersetzung von 2:1. Im Rahmen der Erfindung sind auch andere Übersetzungen möglich. Der Fachmann kennt vergleichbare Flaschenzüge für Hebezeuge.

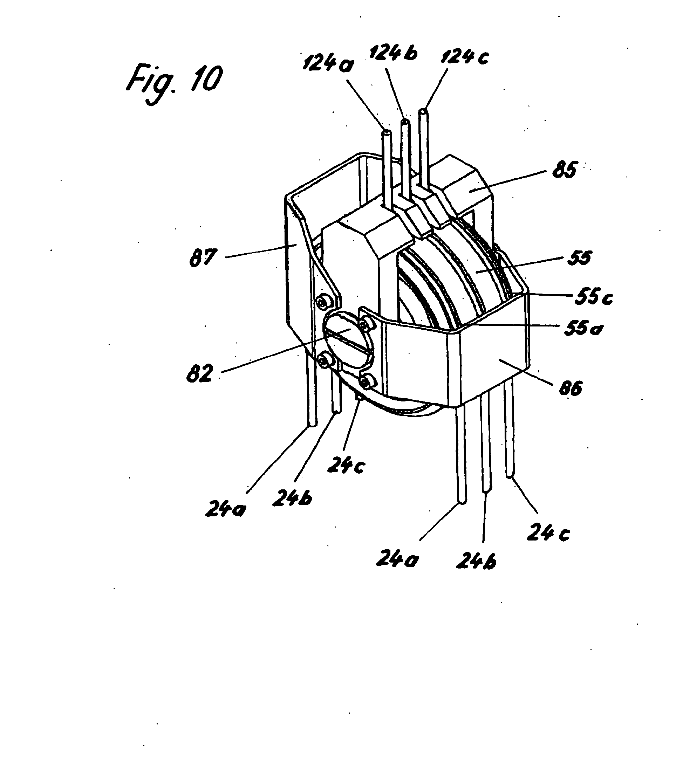

In Fig.10 sieht man Details der Rolle 55 des Flaschenzugs 52, die drei parallele Führungsrillen 55a, 55b und 55c aufweist, um die drei Seile aus dem Seilzug 24a, 24b und 24c darin aufzunehmen. Die Rolle 55 trägt einen Rollenbügel 85 und dieser wiederum ist mit den Halteseilzügen 124a, 124b und 124c verbunden, welche die Zugkraft z.B. zum Bügel 207 (Fig.7) oder zum Gleitschuh 23 (Fig.8) übertragen.

In Fig.11 sieht man einen erfindungsgemäss ausgeführten Stativaufbau mit einem Mikroskop als Last G an einem Schwenkträger 79, der von einem integriert aufgebauten Parallelogrammträger 2b gehalten ist. Der Träger 2b ist an einer Lagerstelle 119 eines Horizontalarms 29 schwenkbar gelagert, der an der Ständersäule 21 gelagert ist. Ein Bügelgriff 88 dient als Transporthilfe und ein Gerätekasten 89 dient neben der Aufnahme von Steuerungen, Stromversorgung, Beleuchtung usw. auch als zusätzliche Kippsicherheitsverbesserung entsprechend dem Aufbau MS 1.

Unter Trägern im Sinne der Patentansprüche sind sowohl einzelne Tragarme als auch Parallelogrammträger oder ähnliche Konstruktionen zu verstehen.

Bezugszeichenliste

1 - Vertikale Achse des vertikalen Ständers
2 - Tragarm bzw. Parallelogrammträger
3 - Arm
4a - Arm
5a - Verbindungsarm
6 a- Parallelogrammträger bzw. Tariergetriebe
7 - Transferelement, vorzugsweise Wagen bzw. Schlitten
8 - Rolle
12 - Tragbauteil bzw. Grundkörper

18a - Federeinheit bzw. Lastausgleichseinheit

19a,b - Spindel

20 - Stativfuss

21 b - Ständersäule

22b - Ausgleichsarm

23 - Gleitschuh

24a,b,c,d - Seilzug

25 - Spindel

26 a,b,c- Umlenkrolle

27 - Drehachse

28a,b - Abstützung

29 - Horizontalarm, könnte auch schräg sein

30 - Rolle

31 - Spindel

33 - Parallelführung

34 - Drehkopplung

35 - Lager

36 - Rolle

37 - Rolle

54 - Lagerstelle

55a,c Flaschenzugrollen

56 - Flaschenzugrollen

79 - Schwenkträger

82 - Nabe

85 - Rollenbügel

86 - Sicherungsbügel

87 - Befestigungsbügel

88 - Bügelgriff

89 - Gerätekasten

119 - Schwenklager

124a,b,c - Halteseilzug

207- Transferelement z.B. Bügel

224 - Anlenkpunkt für Seilzug 24

307 - Wange des Wagens 7

308b - Führung für Wagen 7

AG,a,b - Ausgleichsgewicht

G - Last bzw. Gewicht des Mikroskops

FA - konstante Ausgleichskraft

OT - Operationstisch

MG - Moment um den Schwenkpunkt des oberen Tragarms des Parallelogrammträgers 2b durch G erzeugt

**Patentansprüche**

1. Stativ mit einer Ständersäule **(21)** und einem in einer Vertikalebene schwenkbaren Tragarm **(2)**, für ein Operationsmikroskop als Last **(G)** und mit einer seilgestützten Gewichtsausgleichskraft **(AG)** zur Balancierung des Tragarms **(2)** in beliebigen Schwenkstellungen, wobei die balancierende Ausgleichskraft über einen Seilzug **(24, 124)** unmittelbar oder mittelbar in einem Abstand von der Ständersäule **(21)** am Tragarm **(2)** angreift, mit einer Umlenkung **(26, 36, 37)** , über die der Seilzug **(24, 124)** in eine Parallelrichtung zur Ständersäule **(21)** umgelenkt ist, **dadurch gekennzeichnet, dass** der Seilzug **(24, 124)** an eine Vorrichtung zur Aufbringung einer konstanten Ausgleichskraft **(AGa; AGb)** angeschlossen ist und als Ausgleichskraft **(AGa; AGb)** ein Ausgleichsgewicht **(AG)** vorgesehen ist, welches innerhalb der Ständersäule **(21)** oder entlang derselben vertikal frei beweglich angeordnet ist.

2. Stativ mit wenigstens einem Horizontalarm **(29)** an einer Ständersäule **(21)**, der den schwenkbaren Tragarm **(2)** trägt, nach Anspruch 1, **dadurch gekennzeichnet, dass** die Umlenkung **(26, 36, 37)** den Seilzug **(24, 124)** wenigstens in die Nähe oder in das Innere der Ständersäule **(21)** bringt.

**3.** Stativ nach Anspruch **1 oder 2, dadurch gekennzeichnet, dass** der tragarmnahe Angriffspunkt für den Seilzug **(24, 124)** an einem Übertragungselement **(5; 8)** eines Tariergetriebes **(6)** oder an einem mit dem Übertragungselement **(5; 8)** gekoppelten Transferelement **(7; 207)** befestigt ist.

**4.** Stativ mit einem Grundkörper **(12),** an dem der Tragarm **(2)** gelagert ist, nach Anspruch 3, **gekennzeichnet**, **dass** das Transferelement als Wagen **(7)** oder als Bügel **(207)** ausgebildet und am Grundkörper **(12)** verschiebbar befestigt ist.

**5.** Stativ nach Anspruch 4, **dadurch gekennzeichnet, dass** ein Übertragungselement **(30)** mittels Spindel **(31)** in seiner Relativposition zum Bügel **(207)** verstellbar ist.

**6.** Stativ nach einem der vorhergehenden Ansprüche, **dadurch gekennzeichnet, dass** das Ausgleichsgewicht **(AG)** an einem Flaschenzug **(52)** hängt.

**7.** Stativ nach Anspruch 3-6, **dadurch gekennzeichnet, dass** an wenigstens einer Stelle des Tragarms **(2)** eine Messanordnung zur Ermittlung der Laständerung vorgesehen ist, die über wenigstens einen elektromotorischen Antrieb die Einstellung des Tariergetriebes **(6a)** steuert.

**8.** Stativ nach einem der vorhergehenden Ansprüche, **dadurch gekennzeichnet, dass** der Seilzug **(24, 124)** wenigstens einmal unterbrochen ist und dass an der Unterbrechungsstelle eine Drehentkopplung angeordnet ist, wobei beim Seilzug **(24, 124)** torsionstolerante Seile verwendet werden und der Seilzug **(24, 124)** durch ein Über- oder Untersetzungsgetriebe unterbrochen ist, wobei das Über- oder Untersetzungsgetriebe durch wenigstens zwei Umlenkrollen **(26)** gebildet ist, die miteinander drehfest verbunden sind, wobei die Rollen **(26a, 26b)** einen unterschiedlichen Durchmesser aufweisen.

**9.** Stativ nach einem der vorhergehenden Ansprüche, **dadurch gekennzeichnet, dass** der Seilzug **(24)** an einem Gleitschuh **(23)** befestigt ist, der an einem mit dem Tragarm **(2)** starr verbundenen Ausgleichsarm **(22)** verschiebbar angeordnet ist und der Gleitschuh **(23)** mittels Spindel **(25)** in seiner Lage entlang des Ausgleichsarms **(22)** verstellbar ist.

**10.** Stativ nach Anspruch 8, **dadurch gekennzeichnet, dass** zwischen dem Tariergetriebe **(6)** und der Umlenkrolle **(26)** ein Wagen **(7)** vorgesehen ist, der gegenüber einer gemeinsamen Basis **(12)** verschiebbar gelagert ist und der Kraftübertragung zwischen dem Seilzug **(24)** und dem Tariergetriebe **(6)** dient.

**Claims**

**1.** Stand having an upright column (21) and a support arm (2) which can be pivoted in a vertical plane for a surgical microscope as a load (G), and having a cable-supported weight equalizing force (AG) for balancing the support arm (2) in any desired pivoted positions, the balancing equalizing force acting via a cable pull (24, 124) on the support arm (2) in a direct or indirect fashion at a distance from the upright column (21), and having a deflecting means (26, 36, 37) via which the cable pull (24, 124) is deflected in a direction parallel to the upright column (21), **characterized in that** the cable pull (24, 124) is connected to an apparatus for applying a constant equalizing force (AGa; AGb) and is provided as equalizing force (AGa; AGb) with an equalizing weight (AG) which is arranged inside the upright column (21) or along the latter such that it can move freely vertically.

**2.** Stand having at least one horizontal arm (29), on an upright column (21), which supports the pivotable support arm (2) according to Claim 1, **characterized in that** the deflecting means (26, 36, 37) brings the cable pull (24, 124) at least into the vicinity or into the interior of the upright column (21).

**3.** Stand according to Claim 1 or 2, **characterized in that** the point of action for the cable pull (24, 124), which is near the support arm, is fastened on a transmission element (5; 8) of a counterbalancing transmission (6), or on a transfer element (7; 207) coupled to the transmission element (5; 8).

**4.** Stand having a basic body (12), on which the support arm (2) is mounted, according to Claim 3, **characterized in that** the transfer element is designed as a carriage (7) or as a bracket (207) and is displaceably fastened on the basic body (12).

**5.** Stand according to Claim 4, **characterized in that** a transmission element (30) can be adjusted in its position relative to the bracket (207) by means of spindles (31).

**6.** Stand according to one of the preceding claims, **characterized in that** the equalizing force (AG) is suspended on a block and tackle (52).

**7.** Stand according to Claims 3-6, **characterized in that** provided at at least one point of the support arm (2) is a measuring arrangement for determining the change in load which controls the setting of the counterbalancing transmission (6a) via at least one electric motor drive.

**8.** Stand according to one of the preceding claims, **characterized in that** the cable pull (24, 124) is interrupted at least once, and **in that** a rotary decoupling means is arranged at the point of interruption, torsion-tolerant cables being used for the cable pull (24, 124), and the cable pull (24, 124) being interrupted by a step-up or reduction gear, the step-up or reduction gear being formed by at least two deflecting rollers (26) which are connected to one another in a rotationally fixed fashion, the rollers (26a, 26b) having a different diameter.

**9.** Stand according to one of the preceding claims, **characterized in that** the cable pull (24) is fastened on a sliding pad (23) which is displaceably arranged on a balance arm (22) rigidly connected to the support arm (2), and the sliding pad (23) can be adjusted in its position along the balance arm (22) by means of spindles (25).

**10.** Stand according to Claim 8, **characterized in that** provided between the counterbalancing transmission (6) and the deflecting roller (26) is a carriage (7) which is mounted such that it can be displaced in relation to a common base (12) and serves to transmit force between the cable pull (24) and the counterbalancing transmission (6).

**Revendications**

**1.** Statif avec une colonne de support (21) et un bras porteur (2) pivotant dans un plan vertical, ayant pour charge (G) un microscope opératoire, et avec une force de compensation de poids (AG) assistée par câble pour l'équilibrage du bras porteur (2) dans des positions de pivotement libres, dans lequel la force de compensation équilibrante est appliquée au bras porteur (2) par l'intermédiaire d'un câble Bowden (24, 124) directement ou indirectement à une certaine distance de la colonne de support (21), avec un renvoi (26, 36, 37), par lequel le câble Bowden (24, 124) est renvoyé dans une direction parallèle à la colonne de support (21), **caractérisé en ce que** le câble Bowden (24, 124) est raccordé à un dispositif d'application d'une force de compensation constante (AGa ; AGb), et comme force de compensation (AGa ; AGb), un contrepoids (AG) est prévu qui est disposé de façon verticalement librement mobile à l'intérieur de la colonne de support (21) ou le long de celle-ci.

**2.** Statif avec au moins un bras horizontal (29) sur une colonne de support (21) qui porte le bras porteur pivotant (2), selon la revendication 1, **caractérisé en ce que** le renvoi (26, 36, 37) amène le câble Bowden (24, 124) au moins à proximité ou à l'intérieur de la colonne de support (21).

**3.** Statif selon la revendication 1 ou 2, **caractérisé en ce que** le point d'attaque proche du bras porteur pour le câble Bowden (24, 124) est fixé à un élément de transmission (5 ; 8) d'un engrenage de tarage (6) ou à un élément de transfert (7 ; 207) couplé à l'élément de transmission (5 ; 8).

**4.** Statif avec un corps de base (12) sur lequel le bras porteur (2) est logé, selon la revendication 3, **caractérisé en ce que** l'élément de transfert est réalisé comme un chariot (7) ou un étrier (207) et fixé au corps de base (12) de façon mobile.

**5.** Statif selon la revendication 4, **caractérisé en ce qu'**un élément de transmission (30) est réglable au moyen d'une broche (31) dans sa position relative par rapport à l'étrier (207).

**6.** Statif selon l'une quelconque des revendications précédentes, **caractérisé en ce que** le contrepoids (AG) est suspendu à un palan (52).

**7.** Statif selon les revendications 3 à 6, **caractérisé en ce qu'**au moins à un endroit du bras porteur (2), un ensemble de mesure pour déterminer la variation de charge est prévu, qui commande par l'intermédiaire d'au moins un entraînement électromoteur le réglage de l'engrenage de tarage (6a).

8.  Statif selon l'une quelconque des revendications précédentes, **caractérisé en ce que** le câble Bowden (24, 124) est interrompu au moins une fois et qu'à l'endroit d'interruption, un découplage rotatif est disposé, des câbles à tolérance de torsion étant utilisés pour le câble Bowden (24, 124) et le câble Bowden (24, 124) étant interrompu par un multiplicateur ou un démultiplicateur, le multiplicateur ou le démultiplicateur étant formé par au moins deux rouleaux de renvoi (26) qui sont reliés ensemble de façon verrouillée en rotation, les rouleaux (26a, 26b) présentant un diamètre différent.

9.  Statif selon l'une quelconque des revendications précédentes, **caractérisé en ce que** le câble Bowden (24) est fixé à un patin (23) qui est disposé de façon mobile sur un bras de compensation (22) raccordé rigidement au bras porteur (2) et le patin (23) est réglable au moyen d'une broche (25) dans sa position le long du bras de compensation (22).

10. Statif selon la revendication 8, **caractérisé en ce qu'**entre l'engrenage de tarage (6) et le rouleau de renvoi (26), un chariot (7) est prévu, qui est logé de façon mobile face à une base commune (12) et sert à la transmission de force entre le câble Bowden (24) et l'engrenage de tarage (6).

Fig. 1

Fig. 2

Fig. 2a

Fig.3

*Fig. 3a*

*Fig. 4*

Fig. 5

Fig. 6

Fig. 7

Fig. 8

36

55

56

54

124 a, b, c

85

224

52

Fig. 9

*Fig. 10*

124a  124b  124c

85

87

55

55c

55a

82

86

24c

24a  24b

24a  24b  24c

Fig. 11